(19) Europäisches Patentamt
European Patent Office
Office européen des brevets

(11) Veröffentlichungsnummer: **0 092 632**
**B1**

# (12) EUROPÄISCHE PATENTSCHRIFT

(45) Veröffentlichungstag der Patentschrift: **04.09.85**

(51) Int. Cl.⁴: **C 07 C 121/75,** C 07 C 154/00, A 01 N 47/06

(21) Anmeldenummer: **83100101.1**

(22) Anmeldetag: **07.01.83**

## (54) Neue Benzonitrilderivate, Verfahren zu ihrer Herstellung und herbicide Mittel.

(30) Priorität: **28.04.82 HU 134082**
**28.04.82 HU 134182**

(43) Veröffentlichungstag der Anmeldung:
**02.11.83 Patentblatt 83/44**

(45) Bekanntmachung des Hinweises auf die Patenterteilung:
**04.09.85 Patentblatt 85/36**

(84) Benannte Vertragsstaaten:
**AT BE DE FR GB IT NL**

(56) Entgegenhaltungen:
**DE - A - 1 542 943**
**DE - A - 1 919 571**
**FR - A - 2 007 923**

**Die Akte enthält technische Angaben, die nach dem Eingang der Anmeldung eingereicht wurden und die nicht in dieser Patentschrift enthalten sind.**

(73) Patentinhaber: **CHINOIN Gyogyszer és Vegyészeti Termékek Gyára RT., To utca 1-5, H-1045 Budapest IV (HU)**

(72) Erfinder: **Schawartz, József, Dipl. Ing. Chem., Kobányai ut 42, H-1101 Budapest (HU)**
Erfinder: **Hornyák Hámori, Mária, Dr. Dipl. Ing. Chem., Deák u. 129, H-1041 Budapest (HU)**
Erfinder: **Sági, Jénos, Szilágyi E. Fasor 65, H-1026 Budapest (HU)**
Erfinder: **Mármarosi Kellner, Katalin, Dipl. Ing. Chem., Ybl Miklós sétány 19, H-2051 Biatorbágy (HU)**
Erfinder: **Radványi Hegedüs, Erzsébet, Bartók Béla ut 82, H-1113 Budapest (HU)**
Erfinder: **Szigeti, Zoltán, Dr., Kutvolgyi ut 69/B3, H-1125 Budapest (HU)**
Erfinder: **Cseh, Edit, Dr., Henslmann u. 3, H-1053 Budapest (HU)**
Erfinder: **Bujtás Tülgyes, Klára, Dr., Fráter tér 5, H-1149 Budapest (HU)**

(74) Vertreter: **Patentanwälte Beetz sen. - Beetz jun. Timpe - Siegfried - Schmitt-Fumian, Steinsdorfstrasse 10, D-8000 München 22 (DE)**

EP 0 092 632 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue Benzonitrilderivate mit herbizider Wirksamkeit, ein Verfahren zu ihrer Herstellung und ihre Verwendung als Wirkstoffe in herbiziden Mitteln.

Die Wirkstoffe entsprechen der allgemeinen Formel II

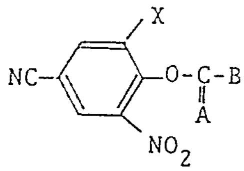

(II)

in der bedeuten:

A Sauerstoff oder Schwefel,

B O-Alkyl, O-Alkenyl, O-Aryl, O-Alkoxyalkyl oder Dimethylamino

und

X Chlor, Brom oder Jod.

Unter Alkyl- oder Alkoxy-Gruppen werden hierbei geradkettige oder verzweigte Alkyl- oder Alkoxy Gruppen mit 1 bis 4 Kohlenstoffatomen verstanden. Der Begriff Aryl umfasst Arylgruppen mit 6 bis 10 Kohlenstoffatomen und bedeutet vorzugsweise Phenyl. Unter dem Begriff Alkenyl sind Alkenylgruppen mit 2 bis 6 Kohlenstoffatomen, vorzugsweise die Allyl-Gruppe, zu verstehen.

Die den erfindungsgemässen Wirkstoffen der allgemeinen Formel II am nähesten stehenden herkömmlichen Wirkstoffe besitzen die allgemeine Formel I

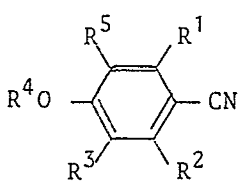

(I)

und sind in der ungarischen Patentschrift 154690 beschrieben (vgl. auch die DE-A-1542943). In der obigen allgemeinen Formel I bedeuten:

$R^1$ Wasserstoff oder Halogen,

$R^2$ Wasserstoff oder Halogen,

$R^3$ Halogen,

$R^4$ Halogen, Acyl oder Alkoxycarbonyl, Alkalimetall oder Erdalkalimetall, Ammonium oder eine organische Aminogruppe,

$R^5$ Wasserstoff, Halogen oder Nitro, mit der Bedingung, dass mindestens einer und höchstens zwei der Substituenten Wasserstoff bedeuten und mit der weiteren Bedingung, dass,

(1) falls $R^1$ und $R^2$ Wasserstoff und $R^3$ und $R^5$ Jod bedeuten, $R^4$ nicht für Wasserstoff, Acetyl, Natrium oder Kalium stehen kann, und,

(2) falls $R^1$ und $R^2$ Wasserstoff und $R^3$ und $R^4$ zugleich Brom oder Chlor bedeuten, $R^4$ nicht für Wasserstoff, Acetyl oder Natrium stehen kann.

Aus der FR-A-2007923 sind ferner Benzonitrilderivate der Formel

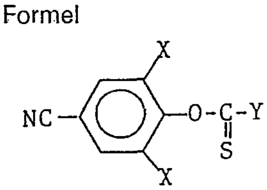

in der X Cl, Br oder J

und

Y F oder Cl

bedeuten.

Der DE-A-1919571 sind Benzonitrilderivate der Formel

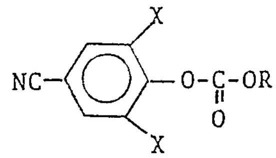

zu entnehmen, in der X Br oder J

und

R Allyl, Tetrahydrofurfuryl, ggf. halogensubstituiertes Phenyl, Benzyl, 2,2,2-Trichlorethyl oder 2-Methoxyethyl bedeuten.

Die Erfindung beruht auf der Feststellung, dass Verbindungen der allgemeinen Formel II als Wirkstoff enthaltende herbizide Mittel im Nachauflaufverfahren zur selektiven Ausrottung von Unkräutern sehr geeignet sind, weil die auf Monokotyledonen und Dikotyledonen ausgeübte Wirkung der Verbindungen der allgemeinen Formel II sehr verschieden ist. Im Nachauflaufverfahren schädigen die Verbindungen der allgemeinen Formel II Monokotyledonen zu 15 bis 25% und Dikotyledonen zu 70 bis 85%.

Das bekannte 3.5-Dibrom-4-hydroxybenzonitril (Bromoxynil) bzw. sein Octansäureester (Brominal) schädigen demgegenüber Monokotyledonen in einem sehr hohen Masse (89%) und Dikotyledonen in gleichem Masse (89%). Die bekannten Verbindungen der allgemeinen Formel I können in einer Dosis von 0,28 bis 0,56 kg/ha und maximal in einer Dosis von 0,56 bis 1,68 kg/ha ohne Schädigung der Kulturpflanzen verwendet werden, während zu einer totalen Ausrottung der Unkräuter die Verbindungen der allgemeinen Formel I in einer Dosis von 2,24 bis 4,48 kg/ha angewandt werden müssen.

Die erfindungsgemässen Wirkstoffe der allgemeinen Formel II können auch noch in einer Dosis von 7 kg/ha zur Unkrautbekämpfung in Mais verwendet werden, ohne die Kulturpflanzen zu schädigen.

Es wurde weiterhin gefunden, dass die Wirkstoffe der allgemeinen Formel II die in Maiskulturen immer grössere Schwierigkeiten verursachenden triazinresistenten Amaranthus- und Chenopodiumarten in viel stärkerem Masse ausrotten als das bekannte Brominal.

Auch die Verbindungen gemäss der FR-A-2007923 sowie der DE-A-1919571 sind demgegenüber weniger wirksam bzw. weniger selektiv.

Die Wirkungsversuche wurden auf Kleinparzellen mit Bestandbehandlung von Mais wie folgt durchgeführt: Vor der Aussaat wurde 25 bis 32 cm tief geackert und danach geeggt; eine Grundbehandlung mit einem herbiziden Mittel wurde nicht vorgenommen. Die Nachauflaufbehandlung wurde im einmonatigen Stadium des Mais mit 300 l Wasser/ha und 0,5-1,0-1,5 kg Wirkstoff/ha durchgeführt. Zur Behandlung wurde ein Mittel mit folgender Zusammensetzung verwendet:

(2-Brom-4-cyano-6-nitrophenyl)-methylcarbonat der Formel IIa

(Gew.-%)

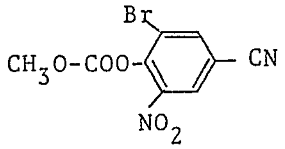 (IIa) 80

Alkylnaphthalinsulfonsäure-Natriumsalz (Wettol NT 1) 3
Ligninsulfonsäure-Natriumsalz (Borresperse B) 5
Siliciumdioxid (Kreide) 12

Die Zerstäubung wurde mit einer Druckdüse bei einem Druck von 2,0 bis 2,5 kg/cm² vorgenommen. Als Standard wurde Brominal 32EC (formuliertes Mittel mit 3.5-Dibrom-4-hydroxybenzonitriloctoat (Wirkstoff B) in einer Dosis von 0,66 kg Wirkstoff/ha verwendet.

Entwicklungsstand der dominierenden Unkräuter während der Behandlung:

| | (cm) |
|---|---|
| Amaranthus retroflexus | 20 |
| Amaranthus chlorostachys | 20 |
| Chenopodium album | 15-20 |
| Hibiscus trionum | 4-6 |
| Stachys annua | 4-8 |
| Malva neglecta, 4-6blättrig | |

Die Versuche wurden 10 Tage nach der Behandlung ausgewertet. Die Ergebnisse sind in Tabelle 1 angegeben. Das als Standard verwendete Brominal verursachte milde phytotoxische Symptome.

Die Bewertung wurde gemäß der EWEC-Skala durchgeführt:

| | Wirksamkeit | |
|---|---|---|
| 1 | ausgezeichnet | (100%) |
| 2 | sehr gut | (98%) |
| 3 | gut | (95%) |
| 4 | ausreichend | (90%) |
| 5 | fraglich | (82%) |
| 6 | nicht ausreichend | (70%) |
| 7 | schlecht | (55%) |
| 8 | sehr schlecht | (30%) |
| 9 | unanwendbar | (0%) |

*Tabelle 1*

| Wirkstoff | Dosis (kg Wirkstoff/ha) | A. retroflexus | A. chlorostachys | C. album | H. trionum | S. annua | M. neglecta |
|---|---|---|---|---|---|---|---|
| B | 0,66 | 5 | 6 | 3 | 5 | 6 | 4 |
| | 0,5 | 3 | 4 | 2 | 3 | 6 | 4 |
| | 1,0 | 2 | 2 | 1 | 3 | 4 | 3 |
| IIa | 1,5 | 2 | 2 | 1 | 3 | 4 | 3 |

Die erfindungsgemässen Wirkstoffe der allgemeinen Formel II sind auch zur Unkrautbekämpfung im Nachauflaufverfahren in zahlreichen Gemüsekulturen wie Erbsen, Zwiebeln, Mohrrüben usw. geeignet.

Mit einem Mittel folgender Zusammensetzung wurden Versuche im Gewächshaus durchgeführt: (2-Brom-4-cyano-6-nitrophenyl)-allylcarbonat der Formel IIb

(Gew.-%)
80

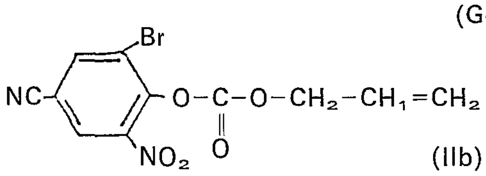 (IIb)

Alkylnaphthalinsulfonsäure-Natriumsalz (Wettol NT 1) 3
Ligninsulfonsäure-Natriumsalz (Borresperse B) 5
Siliciumdioxid (Kreide) 12

Als Standard wurde Brominal 32EC (formuliertes Mittel mit 3.5-Dibrom-4-hydroxybenzonitriloctoat (Wirkstoff B)) verwendet.

Die Ergebnisse sind in Beispiel 6 angegeben.

Es wurden ausführliche Versuche durchgeführt, um zu bestimmen, ob die in den Freilandversuchen beobachteten Unterschiede zwischen der Wirkung und Selektivität von Bromoxynil und den erfindungsgemässen Wirkstoffen der Formel II auch im Wirkungsmechnismus bzw. in der Wirkungsweise zu beobachten sind. Hierzu wurde die Wirkung der Verbindungen auf die Aktivität der Photosynthese geprüft.

Von den dihalogensubstituierten Hydroxybenzonitril-Derivaten (3.5-Dijod-4-hydroxybenzonitril (Ioxynil); 3.5-Dibrom-4-hydroxybenzonitril (Bromoxynil)) ist allgemein anerkannt, dass ihr primärer Angriffspunkt in der photosynthetischen Elektronentransportkette liegt. Des weiteren wurden der einfache Wachstumsprozess (Verlängerungsprozess) und die Membranfunktion (aktiver $K^+$-Influx; Elektrolyt-Efflux) untersucht.

Im Wachstumstest (Verlängerung der Wurzel bei Salat-Keimpflanzen) wurde nachgewiesen, dass die Dibrom-Derivate eine ähnliche Wirkung wie pflanzenwachstumsregulierende Hormone aufweisen: Bei niedriger Konzentration stimulieren sie die Verlängerung der Wurzel, bei höherer Konzentration tritt eine Hemmung auf. Eine ähnliche wachstumsregulierende Wirkung wurde bisher nur bei den 2.4-Diestern vom Typ des Bromoxynils nachgewiesen (Heywood, Chem. and Ind. 1966, 1946-1952). Die Wirkung von (2-Brom-4-cyano-6-nitrophenyl)-alkylcarbonaten ist unterschiedlich; nach anfänglicher Unwirksamkeit kann eine hemmende Wirkung beobachtet werden; eine Wachstumsregulierung findet nicht statt (vgl. Beispiel 3).

Anhand des Vergleichs der Ergebnisse der

Wachstums- und Membranfunktionstests kann festgestellt werden, dass der Grund für die im biologischen Wirkungsspektrum der erfindungsgemässen Wirkstoffe und der bekannten Benzonitril-Herbizide vorhandenen Unterschiede darin liegt, dass ihr Eindringen und ihre Translokation in der Pflanze verschieden sind.

Aufgrund der Ergebnisse der Untersuchung der photosynthetischen Aktivität an mono- und dikotylen Testpflanzen, aufgrund der an intakten Pflanzen gemessenen In-vivo-$CO_2$-Bindung und der an isolierten Chloroplasten durchgeführten Dichlorphenol-Indophenol-(DCPIP)-Reduktion kann folgendes festgestellt werden:

(1) Die Dibrom-Verbindungen wirken in vivo und in intakten Pflanzen gleichmässig, und zwar auf Dikotyledonen stark und auf Monokotyledonen mittelmässig stark;

(2) die brom-nitro-substituierten Verbindungen wirken auf Dikotyledonen gleichmässig und auf Monokotyledonen in geringerem Masse, wie Bromoxynil (Beispiel 4).

Aufgrund von Labortests mit Messung der Photosynthetischen Aktivität ist die Selektivität der brom-nitro-substituierten Verbindungen zwischen den Mono- und Dikotyledonen grösser als bei den Dibrom-Verbindungen.

Die erfindungsgemässen Wirkstoffe der allgemeinen Formel II können zum Pflanzenschutz in Form von Mitteln wie benetzbaren Pulvern zum Einstäuben, Pasten, Emulsionskonzentraten u.dgl. verwendet werden. Die Mittel enthalten neben dem Wirkstoff übliche feste oder flüssige Träger und/oder Verdünnungsmittel, grenzflächenaktive Mittel und andere Hilfsmittel.

Die erfindungsgemässen Mittel können 0,1 bis 90 Gew.-% Wirkstoff enthalten. Die Mittel können bei der Verwendung auf die gewünschte Konzentration verdünnt werden.

Die Erfindung betrifft ferner ein Verfahren zur Unkrautbekämpfung in Mais-, Getreide- und Gemüsekulturen im Nachauflaufverfahren. Das erfindungsgemässe Verfahren ist dadurch gekennzeichnet, dass die Unkrautbekämpfung in den genannten Kulturen im Nachauflaufverfahren mit einem 0,1 bis 90 Gew.-% Wirkstoff der allgemeinen Formel II enthaltenden Mittel in einer Dosis von 1 bis 4 kg Wirkstoff/ha durchgeführt wird.

Die Verbindungen der allgemeinen Formel II können durch Umsetzung einer Verbindung der allgemeinen Formel III

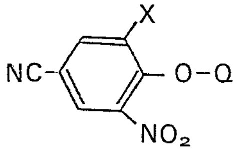
(III)

in der
Q eine tertiäre Base und
X Chlor, Brom oder Jod bedeuten,
mit einem Kohlensäure-Derivat der allgemeinen formel IV

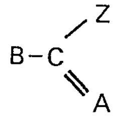
(IV)

in der
A und B die obige Bedeutung besitzen und
Z Chlor oder Brom bedeutet,
hergestellt werden.

Die Reaktion wird vorzugsweise in Gegenwart eines Lösungsmittels bei 20-60° C durchgeführt.

Die Erfindung wird anhand der folgenden Beispiele näher erläutert.

*Beispiel 1:*

*Herstellung von (2-Brom-4-cyano-6-nitrophenyl)-methylcarbonat:*

121,6 g 3-Brom-4-hydroxy-5-nitrobenzonitril werden in 650 ml Benzol aufgenommen und mit 73 ml Triethylamin versetzt. Zu der Lösung werden 44 ml Chlorameisensäuremethylester in 75 ml Benzol während 30 Minuten bei 20-25° C zugetropft. Das Gemisch wird 2 Stunden lang bei 20-25° C und eine Stunde bei 55-60° C gerührt. Danach wird das Gemisch abgekühlt, mit 100 ml Wasser verdünnt und getrennt; die Benzolphase wird mit 100 ml Wasser gewaschen. Das Waschwasser wird mit 100 ml Wasser gewaschen und dann mit 50 ml Benzol extrahiert. Die vereinigten Benzolphasen werden über geglühtem Natriumsulfat getrocknet und mit Aktivkohle entfärbt. Nach Filtration wird die Lösung auf die Hälfte eingeengt; unter Rühren wird 1 l Isopropylether zugegossen. Die ausgeschiedene Kristallmasse wird eine Stunde in Eiswasser gerührt, mit kaltem Methanol gewaschen und getrocknet. Es werden 128 g (85%) Produkt erhalten (Ausbeute 85%). F. 118-120° C.

Auf die gleiche Weise werden aus den entsprechenden Verbindungen der allgemeinen Formeln III und IV die folgenden Verbindungen der allgemeinen formel II hergestellt:

(2-Chlor-4-cyano-6-nitrophenyl)-ethylcarbonat; Ausbeute 87%; F. 89° C;
(2-Jod-4-cyano-6-nitrophenyl)-ethylcarbonat; Ausbeute 87%; F. 112° C;
(2-Brom-4-cyano-6-nitrophenyl)-isopropylcarbonat; Ausbeute 87%; F. 79° C;
(2-Brom-4-cyano-6-nitrophenyl)-isobutylcarbonat; Ausbeute 80%; F. 75° C;
(2-Brom-4-cyano-6-nitrophenyl)-ethylcarbonat; Ausbeute 87,6%; F. 112° C.

*Beispiel 2:*

*Vergleichsversuche mit der Verbindung der Formel IIa und Bromoxynil sowie Brominal im Gewächshaus:*

Die Testpflanzen wurden in Sand in Zuchttöpfen (168 ml) kultiviert und mit einer wässerigen Suspension der formulierten Mittel durch Spritzen behandelt (2 kg Wirkstoff/ha). 14 Tage nach der Behandlung wurde die Wirkung bewertet. In Tabelle 2 ist das Gewicht der Pflanzen in Prozent der Kontrollwerte angegeben. Je kleiner die Zahlenwerte, desto höher die Wirksamkeit der Verbindungen.

*Tabelle 2*

| Testpflanze | Verbindung der Formel IIa (50 WP) | Bromoxynil (32EC) | Brominal (24EC) |
|---|---|---|---|
| Mais | 100 | 67 | 68 |
| Weizen | 100 | 68 | 70 |
| Roggen | 100 | 0 | — |
| Kolbenhirse | 100 | 26 | 26 |
| Erbse | 71 | 0 | 0 |
| Gartenrittersporn | 0 | 0 | — |
| Senf | 0 | 0 | 0 |
| Gartenkresse | 0 | 0 | — |
| Flachs | 0 | 0 | — |
| Mariendistel | 0 | 0 | — |
| Sonnenblume | 0 | 0 | — |
| Sauerampfer | 0 | 0 | — |
| Tomate | 0 | 0 | — |
| Knöterich | 10 | 10 | 11 |
| Fuchsschwanz | 18 | 20 | 20 |

*Beispiel 3*

*Wachstumstest an Salatwurzeln:*

Im Dunkeln bei 25° C zum Keimen gebrachte Pflänzchen von Lactuca sativa mit 5 mm Wurzeln wurden in eine den zu untersuchenden Wirkstoff enthaltende $CaSO_4$-Lösung umgesetzt (Petrischale, 4 ml; 15 Pflanzen). Die Pflanzen wurden danach 48 Stunden lang (Durchschnittstemperatur: 23° C) einer veränderlichen Belichtung (14 Stunden Licht, 10 Stunden Dunkelheit) ausgesetzt. Es wurde das Wachstum der Wurzeln gemessen. Die Ergebnisse sind in Tabelle 3 in Prozent der Kontrollwerte angegeben. Je kleiner die Zahlenwerte, desto höher die Wirksamkeit.

*Tabelle 3*

| Wirstoff | $10^{-6}$M | $10^{-5}$M | $10^{-4}$M |
|---|---|---|---|
| Erfindungsgemässe Wirkstoffe: | | | |
| (2-Brom-4-cyano-6-nitrophenyl)-methylcarbonat | 90,4 | 72,9 | 17,9 |
| (2-Brom-4-cyano-6-nitrophenyl)-ethylcarbonat | 90,5 | 87,7 | 3,7 |
| (2-Brom-4-cyano-6-nitrophenyl)-phenylcarbonat | | 88,7 | |
| (2-Brom-4-cyano-6-nitrophenyl)-allylcarbonat | | 55,1 | |
| (2-Brom-4-cyano-6-nitrophenyl)-methylcarbonat | 90,4 | 72,9 | 17,9 |
| Herkömmliche Wirkstoffe (Vergleichsversuche): | | | |
| 3,5-Dibrom-4-hydroxybenzonitril | 140,6 | 96,6 | 7,0 |
| 3-Brom-4-hydroxy-5-nitrobenzonitril | 88,8 | 95,4 | 10,1 |
| (2,6-Dibrom-4-cyanophenyl)-ethylcarbonat | 149,6 | 91,9 | 35,3 |
| 2,6-Dibrom-4-cyanophenol | 140,6 | 96,6 | 7,0 |
| (2,6-Dibrom-4-cyanophenyl)-phenylcarbonat | | 92,3 | |
| (2,6-Dibrom-4-cyanophenyl)-allylcarbonat | | 79,0 | |
| (2,6-Dibrom-4-cyanophenyl)-methylcarbonat | 128,0 | 86,0 | 34,0 |

*Beispiel 4:*

*Messung der In-vivo-$CO_2$-Bindung bei Weizen und Spinat (Wirkstoffkonzentration $3 \cdot 10^{-5}$M):*

Die In-vivo-$CO_2$-Bindung der Blätter wurde mit $^{14}CO_2$ nach der Methode von Sárvári et al. (Physiol. Plant. *36*, 187-192 (1976)) bestimmt. Nach Beendigung der $CO_2$-Bindung wurde die Radioaktivität der durch Wärmebehandlung abgetöteten Blätter nach dem Flüssigkeitsszintillationsverfahren gemessen. Die Ergebnisse sind in Tabelle 4 in Prozent der Kontrollwerte angegeben, wobei niedrigere Zahlenwerte einer stärkeren Inhibitionswirkung entsprechen.

*Tabelle 4*

| Wirstoff | Spinat (Dikotyledon) | Weizen (Monokotyledon) |
|---|---|---|
| Erfindungsgemässe Wirkstoffe: | | |
| (2-Brom-4-cyano-6-nitrophenyl)-methylcarbonat | 11,0 | 91,0 |
| (2-Brom-4-cyano-6-nitrophenyl)-phenylcarbonat | 6 | 69 |
| (2-Brom-4-cyano-6-nitrophenyl)-allylcarbonat | 11 | 93 |
| (2-Brom-4-cyano-6-nitrophenyl)-phenylcarbonat | 6,0 | 69,0 |
| (2-Brom-4-cyano-6-nitrophenyl)-methylcarbonat | 11,0 | 91,0 |
| (2-Brom-4-cyano-6-nitrophenyl)-allylcarbonat | 11,0 | 93,0 |
| Herkömmliche Wirkstoffe (Vergleichsversuche): | | |
| 3,5-Dibromhydroxybenzonitril | 10,7 | 47,4 |
| (2,6-Dibrom-4-cyanophenyl)-methylcarbonat | 18,6 | 17,0 |
| (2,6-Dibrom-4-cyanophenyl)-allylcarbonat | 4,2 | 37,5 |
| 2,6-Dibrom-4-cyanophenol | 10,7 | 47,4 |
| (2,6-Dibrom-4-cyanophenyl)-phenylcarbonat | 1,0 | 25,0 |

Aus den Ergebnissen von Tabelle 4 geht neben der Feststellung, dass Weizen generell widerstandsfähiger als Spinat ist, hervor, dass die erfindungsgemässen Wirkstoffe Dikotyledonen etwa im gleichen Ausmass wie die Vergleichsverbindungen schädigen, jedoch gegenüber Monokotyledonen eine erheblich höhere Selektivität besitzen.

*Beispiel 5:*

*Herstellung von (2-Brom-4-cyano-6-nitrophenyl)-phenylcarbonat:*

121,5 g 3-Brom-4-hydroxybenzonitril werden in 650 ml Benzol aufgenommen und mit 73 ml Triethylamin versetzt. Zu der Lösung werden 44 ml Chlorameisensäurephenylester in 75 ml Benzol während 30 Minuten bei 20-25° C zugetropft. Das Gemisch wird 2 Stunden lang bei 20-25° C und eine Stunde bei 55-60° C gerührt. Danach wird das Gemisch abgekühlt, mit 100 ml Wasser verdünnt und getrennt; die Benzolphase wird mit 100 ml Wasser gewaschen. Das Waschwasser wird mit 100 ml Wasser gewaschen und dann mit 50 ml Benzol extrahiert.

Die vereinigten Benzolphasen werden über geglühtem Natriumsulfat getrocknet und mit Aktivkohle entfärbt. Nach Filtration wird die Lösung auf die Hälfte eingeengt und unter Rühren mit 1 l Isopropylether versetzt. Die ausgeschiedene Kristallmasse wird eine Stunde in Eiswasser gerührt, filtriert, mit kaltem Methanol gewaschen und getrocknet.

Ausbeute. 92%; F. 86° C

Auf die gleiche Weise werden die folgenden Verbindungen der allgemeinen Formel II hergestellt:

(2-Brom-4-cyano-6-nitrophenyl)-allylcarbonat
Ausbeute 87%; F. 87° C;

(2-Brom-4-cyano-6-nitrophenyl)-2-methoxyethylcarbonat
Ausbeute 85%; F. 100° C;

6-(2-Brom-4-cyano-6-nitrophenyl)-5-ethylcarbonat
Ausbeute 83%; F. 109° C;

O-(2-Brom-4-cyano-6-nitrophenyl)-N.N-dimethylthiocarbonat
Ausbeute 95%; F. 135° C;

(2-Chlor-4-cyano-6-nitrophenyl)-phenylcarbonat
Ausbeute 92%; F. 89° C;

(2-Brom-4-cyano-6-nitrophenyl)-phenylcarbonat
Ausbeute 92%; F. 86° C.

*Beispiel 6:*

*Vergleichsversuche mit der Verbindung der Formel IIb und Bromoxynil im Gewächshaus:*

Die Versuche wurden auf die in Beispiel 2 beschriebene Weise durchgeführt.

In Tabelle 5 ist das Gewicht der Pflanzen in Prozent der Kontrollwerte angegeben.

*Tabelle 5*

| Testpflanze | Verbindung der Formel IIb (50 WP) (2 kg Wirkstoff/ha) | Bromoxynil (32EC) (2 kg Wirkstoff/ha) |
|---|---|---|
| Mais | 100 | 67 |
| Weizen | 100 | 68 |
| Fuchsschwanz (Amaranthus retroflexus) | 18 | 20 |
| Weisser Senf (Sinapis alba) | 0 | 0 |
| Kolbenhirse (Setaria italica) | 26 | 26 |
| Knöterich (Polygonum lagratifilium) | 10 | 10 |

*Beispiel 7:*

Von Verbindungen der Formel II wird folgende WP-Formulierung hergestellt:

80 g Wirkstoff werden vorhomogenisiert; dann werden

3 g Alkylnaphthalinsulfonsäure-Natriumsalz (Wettol NT 1),

5 g Ligninsulfonsäure-Natriumsalz (Borresperse B) mit niedrigem pH-Wert und

12 g Siliciumdioxid (Kreide)

zugegeben, wonach auf unter 10 µm gemahlen wird.

*Beispiel 8:*

Nach dem Verfahren von Beispiel 7 wird eine WP-Formulierung mit folgender Zusammensetzung hergestellt:

85 g Wirkstoff, 4 g Alkylnaphthalinsulfonsäure-Natriumsalz,

5 g Ligninsulfonsäure-Natriumsalz, 1 g Harnstoff, 5 g Natriumsilicat.

*Beispiel 9:*

Von Verbindungen der Formel II wird wie folgt eine emulgierbare Suspension hergestellt:

50 g Wirkstoff werden homogenisiert; dann werden

5 g Alkansulfonsäure-Calciumsalz (Hoe S 1557) und

5 g Fettalkoholpolyglycolether (Genapol O-050) zugegeben, wonach in einer Kugelmühle gemahlen wird.

*Beispiel 10:*

Aus 50 g Wirkstoff, 4 g Atlox 4851 (Gemisch von anionischen und nichtionischen Tensiden), 6 g Atlox 3400, 1 g organophilem Bentonit und 39 g Solventnaphtha wird nach dem Verfahren von Beispiel 9 eine emulgierbare Suspension hergestellt.

## Patentansprüche

1. Benzonitrilderivate der allgemeinen Formel II

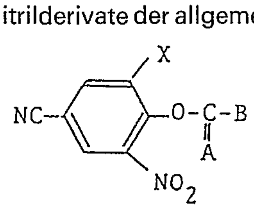

(II)

in der bedeuten:

A Sauerstoff oder Schwefel

B O-Alkyl, O-Alenyl, O-Aryl, O-Alkoxy, O-Alkoxyalkyl oder Dimethylamino und

X Chlor, Brom oder Jod.

2. (2-Brom-4-cyano-6-nitrophenyl)-methylcarbonat.

3. (2-Brom-4-cyano-6-nitrophenyl)-phenylcarbonat.

4. Verfahren zur Herstellung von Verbindungen der allgemeinen Formel II nach einem der Ansprüche 1 bis 3, dadurch gekennzeichnet, dass eine Verbindung der allgemeinen Formel III

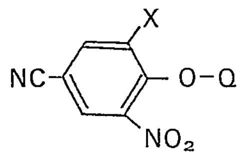

(III)

in der

Q eine tertiäre Base und

X Chlor, Brom oder Jod

bedeuten,

mit einem Kohlensäure-Derivat der allgemeinen Formel IV

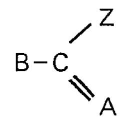

(IV)

mit

A und B wie in Anspruch 1 und

Z=Chlor oder Brom

umgesetzt wird.

5. Herbizide Mittel, gekennzeichnet durch 0,1 bis 90 Gew.-% mindestens einer Verbindung der allgemeinen Formel II nach einem der Ansprüche 1 bis 3 als Wirkstoff neben üblichen Hilfs- und/oder Trägerstoffen.

6. Herbizide Mittel nach Anspruch 5, gekennzeichnet durch (2-Brom-4-cyano-6-nitrophenyl)-methylcarbonat als Wirkstoff.

7. Herbizide Mittel nach Anspruch 5, gekennzeichnet durch (2-Brom-4-cyano-6-nitrophenyl)-phenylcarbonat als Wirkstoff.

8. Verfahren zur selektiven Unkrautbekämpfung in Mais-, Getreide- und Gemüsekulturen, gekennzeichnet durch Anwendung eines Mittels nach einem der Ansprüche 5 bis 7 in einer Dosis von 1 bis 4 kg Wirkstoff/ha im Nachauflaufverfahren.

## Claims

1. Benzonitrile derivatives of the general formula II

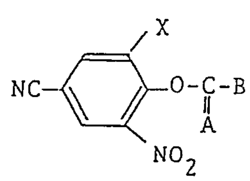

(II)

wherein

A stands for oxygen or sulfur,

B stands for O-alkyl, O-alkenyl, O-alkoxy, O-alkoxyalkyl, or dimethylamino, and

X stands for chloro, bromo, or iodo.

2. (2-Bromo-4-cyano-6-nitrophenyl) methylcarbonate.

3. (2-Bromo-4-cyano-6-nitrophenyl) phenylcarbonate.

4. A process for preparing compounds of the general formula II according to one of claims 1 to 3, characterized in that a compound of the general formula III

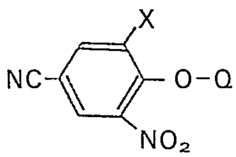

(III)

wherein Q stands for a tertiary base and

X is chloro, bromo, or iodo.

is reacted with a carbonic acid derivative of the general formula IV

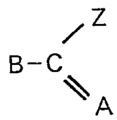

(IV)

wherein

A and B are as in claim 1 and

Z stands for chloro or bromo.

5. A herbicidal composition characterized by 0.1 to 90% by weight of at least one compound of the general formula II according to one of claims 1 to 3 as active ingredient, besides customary auxiliary and/or carrier substances.

6. Herbicidal composition according to claim 5, characterized by (2-bromo-4-cyano-6-nitrophenyl) methylcarbonate as active ingredient.

7. Herbicidal composition according to claim 5, characterized by (2-bromo-4-cyano-6-nitrophenyl) phenylcarbonate as active ingredient.

8. A method for selective weed control in corn, cereal, and vegetable cultures, characterized by the use of a composition according to one of claims 5 to 7 in a dosage of 1 to 4 kg of active ingredient per hectare by post-emergence application.

## Revendications

1. Dérivés de benzonitrile de formule générale II

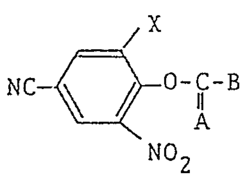

(II)

où

A représente un oxygène ou un soufre,

B représente un O-alcoyle, O-alcényle, O-aryle, O-alcoxy, O-alcoxyalcoyle ou diméthylamino et

X représente un chlore, un brome ou un iode.

2. (2-Bromo-4-cyano-6-nitrophényl)-méthylcarbonate.

3. (2-Bromo-4-cyano-6-nitrophényl)-phénylcarbonate.

4. Procédé de préparation de composés de formule générale II selon l'une des revendications 1 à 3, caractérisé en ce qu'on fait réagir un composé de formule générale III

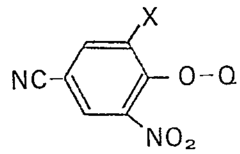

(III)

où Q représente une base tertiaire et

X représente un chlore, un brome ou un iode, avec un dérivé d'acide carbonique de formule générale IV

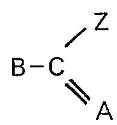

(IV)

où A et B sont tels que dans la revendication 1 et

Z représente un chlore ou un brome.

5. Agents herbicides caractérisés par de 0,1 à 90% en poids d'au moins un composé de formule générale II selon l'une des revendications 1 à 3 comme matière active outre les additifs et/ou supports habituels.

6. Agents herbicides selon la revendication 5, caractérisés par le (2-bromo-4-cyano-6-nitrophényl)-méthylcarbonate comme matière active.

7. Agents herbicides selon la revendication 5, caractérisé par le (2-bromo-4-cyano-6-nitrophényl)-phénylcarbonate comme matière active.

8. Procédé de lutte sélective contre les mauvaises herbes dans les cultures de maïs, de céréales et de légumes, caractérisé par l'application d'un agent selon l'une des revendications 5 à 7 à une dose de 1 à 4 kg de matière active par hectare dans le procédé de postlevée.